# EUROPEAN PATENT SPECIFICATION

(11) **EP 4 176 260 B1**
(45) Date of publication and mention of the grant of the patent: **25.03.2026**
(21) Application number: 21737832.2
(22) Date of filing: 29.06.2021
(51) Int. Cl.: G01N 33/497, G01N 33/00

(54) **TEST SYSTEM**
TESTSYSTEM
SYSTÈME DE TEST

(30) Priority: 02.07.2020 SE 2050823
(43) Date of publication of application: 10.05.2023
(73) Proprietor: Automotive Coalition for Traffic Safety, Inc., Ashburn, VA 20147 (US)
(72) Inventor: GRANSTAM, Mathias, 725 94 Västerås (SE); HESSMAN PETTERSSON, Karl-Adam, 723 41 Västerås (SE); HOLMQVIST, Niclas, 732 42 Västerås (SE)
(74) Representative: Zacco Sweden AB
(86) International application number: PCT/SE2021/050647
(87) International publication number: WO 2022/005381

(56) References cited:
- WO-A1-2019/115473
- CN-A- 106 645 587
- CN-B- 105 021 777
- SE-C2- 531 741
- US-A- 3 665 748
- US-A- 4 407 152
- US-A- 5 728 927
- US-A- 5 731 508
- US-A1- 2018 252 699
- US-B2- 7 845 206

## Description

### Field of invention

The present invention relates to a system for detection and quantification of substances in a gas mixture, as well as a system and method for generating a gas mixture. The present invention also relates to methods of testing performance of alcohol breath analyzer units.

### Background

Drunk driving is a global problem that accounts for thousands of lives annually in US alone, similar numbers have been recorded in Europe. Most countries today have a limit for alcohol content in the breath allowed during driving. To enforce this limit alcohol breath analyzers are important tools. The current state of the art alcohol breath analyzers requires a forced exhalation received by a mouthpiece. Recently there has been a development for a novel type of alcohol breath analyzers that do not require a mouthpiece or a forced exhalation. Such alcohol breath analyzers are more user friendly and therefore more likely to be accepted by the general public. One of the challenges with such a device or system without a mouthpiece is that the amount of available gas from the exhalation is much lower. The detection limit of the analyzer therefore needs to be lower than for current devices, they also must be reliable. An alcohol breath analyzer also needs to be able to detect the breath alcohol content at various environmental conditions. Therefore, a test system further needs to be able to test alcohol breath analyzers at different conditions, such as different temperatures and different relative humidities.

US 2018/0252699 discloses a metrological bench for calibrating breath alcohol testers, the metrological bench is adapted to carry out a method involving delivering to the tester a sample of gas that varies in terms of ethanol concentration, CO₂ concentration, flow rate, pressure and temperature in a manner equivalent to the variances exhibited by a human.

US 2011/0107813 discloses a breath test analyzer with vapor including ethyl alcohol including a heated thermal mass to heat an inlet passage.

J. Ljungblad "High performance breath alcohol analysis" Malardalen University Press Dissertations No. 240 2017 discloses alcohol breath analyzers designed for operations without a mouthpiece. Experiments show that given enough time and analyzer resolution, passive alcohol detection systems are feasible.

US 4 407 152 A discloses a wet gas simulator apparatus where the accuracy of the gas composition is controlled by controlling the temperature of the water-ethanol mixture, without further devices to verify it.

There is a need for improvement of the systems and methods that generates a gas mixture that mimics a human exhalation, wherein the gas mixture can comprise a controlled amount of alcohol. The system could be used to the test the performance of alcohol breath analyzers Another need in the area is a system that enables testing of alcohol breath analyzers at varying environmental conditions.

### Summary

The object of the invention is to obtain an improved system and method for generation of gas that mimics the human exhalation, wherein the system can be used to the test the performance of alcohol breath analyzers. This is achieved by the system in claim 1 and the method in claim 13. Further embodiments are defined in the dependent claims.

### List of figures

Figure 1a is a schematic illustration of one embodiment according to the invention;
Figure 1b is a schematic illustration of a gas flow path chart according to the invention;
Figure 2 is a schematic illustration of one embodiment according to the invention;
Figure 3 is a schematic illustration of one embodiment according to the invention;
Figure 4 is a schematic illustration of a method according to the invention;
Figure 5 is a schematic illustration of a method according to the invention; and
Figure 6a and b are schematic illustrations of methods according to the invention.

### Detailed description

Terms such as "top", "on top", "bottom", upper", lower", "below", "above" etc. are used merely with reference to the geometry of the embodiment of the invention shown in the drawings and/or during normal operation of the described system and its components are not intended to limit the invention in any manner.

The aim of an alcohol breath analyzer is to measure (or test) the alcohol concentration in breath from a human. The output (result) from such a test can be used for example to determine whether the person can drive a car or not depending on the concentration of alcohol in the breath. For such a task an alcohol breath analyzer needs to be reliable and measure the correct alcohol concentration in the breath. Therefore, the performance, i.e. the margin of error for such measurements, of alcohol breath analyzers are tested in a test system. A test system may be arranged to test the performance of individual alcohol breath analyzers.

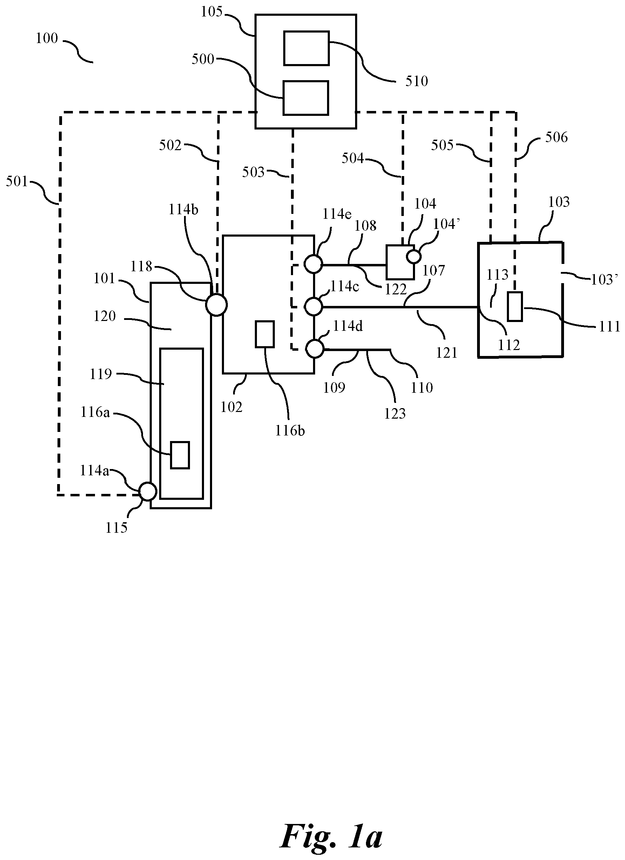

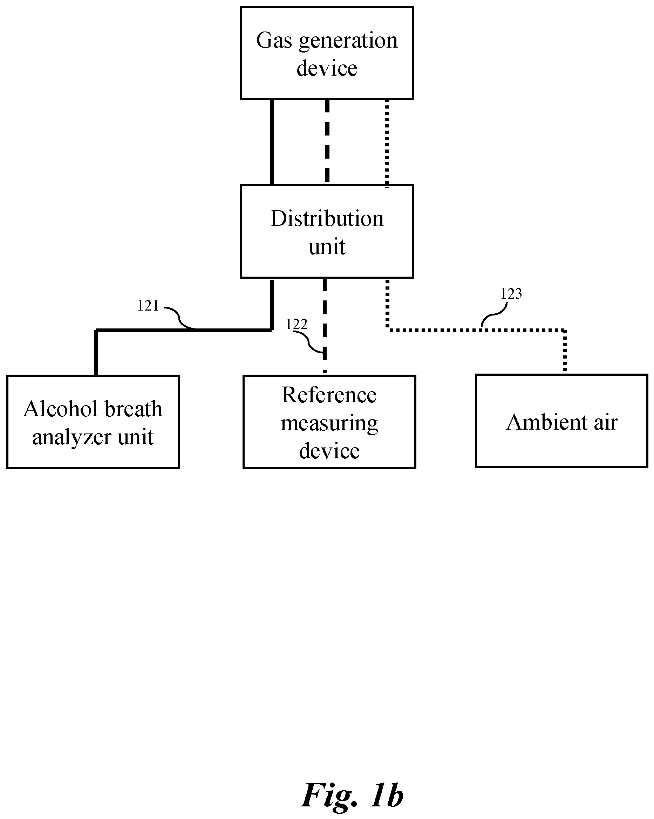

In one aspect of the invention there is a system 100 for testing alcohol breath analyzer units 111. Such a system is schematically illustrated in Figure 1a. The system 100 comprises an alcohol breath gas generation device 101, a distribution unit 102, a test chamber 103, a reference measuring device 104, and a control and registration unit 105. The alcohol breath gas generation device 101 is arranged to generate a gas mixture comprising alcohol, or a gas mixture without alcohol. The gas generation procedure is explained in further detail below. The gas mixture may be a mixture of for example air, carbon dioxide (CO₂), and, not optionally, water and alcohol vapor so that it corresponds to a human exhalation containing alcohol vapor. The alcohol breath gas generation device 101 is in fluid communication with the distribution unit 102 that is arranged downstream the alcohol breath gas generation device 101. The distribution unit 102 is arranged to distribute the gas generated by the alcohol breath gas generation device 101 further downstream the system 100, via a first gas flow tube 107, a second gas flow tube 108, and a third gas flow tube 109. The first gas flow tube 107 is arranged to distribute gas from the distribution unit 102 to the test chamber 103, the second gas flow tube 108 is arranged to distribute gas from the distribution unit 102 to the reference measuring device 104, and the third gas flow tube 109 is arranged to distribute gas from the distribution unit 102 to a waste outlet 110. The system 100 provides for three functional gas flow paths, schematically illustrated in the gas flow path chart of fig 1b. The first gas flow path 121 functionally provides the gas generated in the gas generator to the alcohol breath analyzer unit, via the distribution unit. The second gas flow path 122 functionally provides the gas generated in the gas generator to the reference measuring device, via the distribution unit. The third gas flow path 123 functionally provides means to flush the system by providing an opening to ambient air, via the distribution unit.

The gas generation device 101, the distribution unit 102, and the first gas flow tube 107 forms a first gas flow path 121 leading from the gas generation device 101 to the test chamber 103. The gas generation device 101, the distribution unit 102, and the second gas flow tube 108 forms a second gas flow path 122 leading from the gas generation device 101 to the reference measuring device 104. The gas generation device 101, the distribution unit 102, and the third gas flow tube 109 forms a third gas flow path 123 leading from the gas generation device 101 to the waste outlet 110. The third gas flow path 123 may, as depicted in figure 1a end in a separate outlet 110 from the distribution outlet 102. Alternatively, representing different embodiments an opening to the ambient air is provided in the test chamber 103, opening 103', or in connection with the reference instrument, reference valve 104' that can provide an opening to the ambient air. The waste outlet 110, the test chamber 103, and the reference measuring device 104 are all arranged downstream the distribution unit 102. Hence, the alcohol breath gas generation device 101 is during use of the system 100 in fluid communication with the distribution unit 102 that is in fluid communication with the test chamber 103, the reference measuring device 104, and the waste outlet 110. In this way gas that is generated by the gas generation device 101 reach the gas distribution unit 102 where it is distributed to one of the three flow tubes (i.e. the first gas flow tube 107, the second gas flow tube 108, or the third gas flow tube 109). The gas that enters the first gas flow tube 107 travels from the gas distribution unit 102 to the test chamber 103, the gas that enters the second gas flow tube 108 travels from the gas distribution unit 102 to the reference measuring device 104, and the gas that enters the third gas flow tube 109 travels from the gas distribution unit 102 to the waste outlet 110.

During use of the test system 100 at least one alcohol breath analyzer unit 111 is placed in the test chamber 103. The test system 100 is arranged to provide a gas mixture that the at least one alcohol breath analyzer unit 111 is exposed to. The alcohol breath gas generation device 101 is arranged to generate said gas mixture during use of the test system 100. The gas mixture can be any mixture of air, carbon dioxide, and non-optionally water and alcohol. In other embodiments not part of the present invention the alcohol in the mixture may be exchanged for another substance such as for example methanol or acetone. In such embodiments the at least one alcohol breath analyzer unit 111 may be exchanged for an analyzer unit configured to measure the substance provided in the gas mixture. Additionally, the reference measuring device is a reference measuring device configured to measure the substance (at least alcohol) provided in the gas mixture.

According to the invention, the gas mixture comprises alcohol and the concentration of alcohol is known, the gas mixture may further have a known concentration of carbon dioxide and/or water.

The system 100 may comprise one or more heaters, such as a first heater 116a and a second heater 116b arranged to heat, or maintain a predetermined temperature of, the gas mixture and/or other components or parts of the system 100. Keeping the gas mixture at a controlled temperature may be important in order to not change the composition of the gas, i.e. if the temperature of the gas varies the composition of the gas mixture may vary. It may for example be important that the temperature of the gas in the system 100 is above the temperature of the dew point of the gas. During use of the system 100 the at least one alcohol breath analyzer unit 111 is arranged to be exposed to the gas mixture. Such an exposure can be used to test the performance of the at least one alcohol breath analyzer unit 111. If the at least one alcohol breath analyzer unit 111 is arranged to be exposed to a gas mixture without alcohol, the at least one alcohol breath analyzer unit 111 may be tested at a zero-level alcohol content. The at least one alcohol breath analyzer unit 111 is arranged to measure the alcohol content, i.e. the alcohol concentration, and/or the carbon dioxide content of the gas with known alcohol concentration in order to test its performance. The at least one alcohol breath analyzer unit 111 may comprise a device for measuring and controlling temperature.

Gas flow tubes 107; 108; 109 are arranged to distribute gas through the system 100. The gas flow tubes 107; 108; 109 may be fabricated in for example a stainless material or a polymer such as polytetrafluoroethylene (PTFE). The exit 112 is arranged to allow gas from the second gas flow tube 107 to enter the test chamber 103. At the exit 112 the inner cross-section of the gas flow tube 107 may be 1-4 cm, or 1/2", so that it mimics the size of an open mouth. Furthermore, between the exit 112 and the alcohol breath analyzer unit 111 there is a gas cloud space 113. The gas cloud space 113 may be arranged to be empty so that no item is placed in between the exit 112 and the alcohol breath analyzer unit 111 to interfere with the gas flow from the exit 112 to the alcohol breath analyzer unit 111. In this way the gas flow may mimic an exhalation from a person. The gas cloud space 113 is a certain volume in the test chamber 103 that has a certain distance, for example 0-30 cm, so that the distance between the exit 112 and the alcohol breath analyzer unit 111 is for example 0-30 cm.

Valves 114a-e are arranged to regulate the flow of gas in the system 100 during use of the system 100. A first valve 114a is arranged at the inlet 115 to the alcohol breath gas generation device 101. A second valve 114b is arranged in between the gas generation device 101 and the distribution unit 102. The distribution unit 102 further comprises three additional valves: a third valve 114c, a fourth valve 114d, and a fifth valve 114e arranged at the first gas flow tube 107, the second gas flow tube 108, and the third gas flow tube 109, respectively. The valves 114a-e may be arranged to enable so that the system 100 has the same, or essentially the same, internal pressure at all parts of the system 100. This may for example be achieved by valves 114a-e with an inner diameter of 0.5-2 cm, or 1/4". It may also be achieved using pneumatic valves inside the system 100, hence that the valves 114a-e are pneumatic.

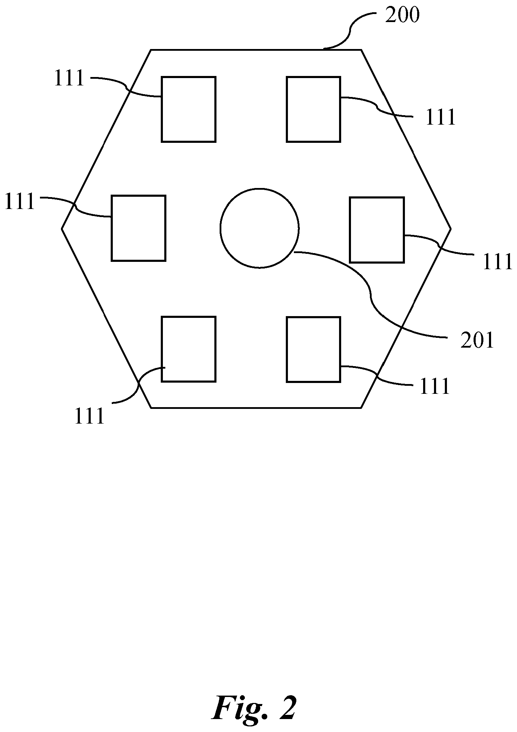

At least one alcohol breath analyzer unit 111 is arranged to be placed in the test chamber 103 during use of the test system 100. In other embodiments the test chamber 103 may hold more than one alcohol breath analyzer unit 111, such as two or more, or three or more, or four or more alcohol breath analyzer units 111, during use of the test system 100. In one embodiment the test chamber 103 comprises a device for changing alcohol breath analyzer 200, schematically illustrated in Figure 2. The device 200 may comprise a revolver mechanism. In embodiments where the test chamber 103 comprises a device for changing alcohol breath analyzer 200 the test chamber may hold several alcohol breath analyzer units 111 that may be tested sequentially so that the device for changing alcohol breath analyzer 200 is arranged to change the alcohol breath analyzer unit 111 that is tested by the system 100. The device for changing alcohol breath analyzer 200 may comprise an axis 201 so that it can be rotated in order to change the alcohol breath analyzer unit 111 that is tested. During testing an alcohol breath analyzer 111 is positioned in front of the gas exit 112. The device for changing alcohol breath analyzer 200 may be positioned so that the distance between the alcohol breath analyzer 111 and the gas exit 112 is the same and remain constant during the testing sequence for all alcohol breath analyzers 111. Hence, the device for changing alcohol breath analyzer 200 may be arranged to maintain an alcohol breath analyzer unit 111 at a predetermined distance from the gas exit 112 during a predetermined time period, during which the alcohol breath analyzer unit 111 may be arranged to be exposed to a gas mixture. In one embodiment the test chamber 103 is a climate chamber so that the test chamber 103 may be arranged to change and/or control the temperature and/or relative humidity (RH) inside the test chamber 103. In an embodiment where the test chamber 103 is a climate chamber the at least one alcohol breath analyzer unit 111 may be arranged to be tested at different relative humidities and/or temperatures, such as at various environmental conditions of ambient temperature and humidity. In one embodiment the test chamber 103 is arranged to provide temperatures between -40 °C to +120 °C, and/or relative humidities (RH) between 10 % and 95 %. The test chamber 103 comprises one opening 103' towards the ambient air. Such an opening 103' may enable that the gas that enters the test chamber 103 at the exit 112 does not stay inside the test chamber 103 and build up a background level but instead exit the test chamber 103 at the opening 103'. The opening 103' may be realized by for example an opening in the wall of the test chamber 103, a filter, a door that can be opened, a valve, etc. The opening 103' may also be controlled by for example the control and registration unit 105.

The waste outlet 110 is arranged downstream the distribution unit 102 and the alcohol breath gas generation device 101. Hence, the distribution unit 102 may be flushed with gas that can exit the system 100 at the waste outlet 110. The waste outlet 110 may be open to the ambient air. In such way the system 100 may be flushed with gas in between testing sequences. Flushing the system 100 via the third gas flow path 123 so that the gas exits the system 100 at the outlet 110 may prevent a background level from building up in the test chamber 103 during the flushing.

The system 100 may be arranged to be heated in order to produce gas with a composition that mimics human breath and/or in order to avoid condensation of the gas inside the system 100. In order to heat the system 100, it may comprise heaters: a first 116a and a second 116b heater. In one embodiment the first heater 116a is arranged at the alcohol breath gas generation device 101, and the second heater 116b is arranged at the distribution unit 102. The gas flow tubes 107; 108; 109 may also be heated, the gas flow tubes 107; 108; 109 may be heated in sections so that the temperature of the gas flow tubes 107; 108; 109 is lower downstream in the system 100 than upstream, in such way condensation of gas inside the gas flow tubes 106; 107; 108; 109 may be avoided. The temperature of the parts of the system 100 may be higher upstream in the system 100 than downstream. The temperature of the gas flow tubes 107; 108; 109 may be stable over time. That the temperatures of the gas flow tubes 107; 108; 109 are stable may enable that the composition of the gas does not vary. In one embodiment the gas distribution unit 102 is arranged to be heated to a temperature of 30-40 °C during use of the system 100, for example the temperature of the gas generation device 101 may be 34 °C, the temperature of the distribution unit 102 may be 36 °C, and the temperature of the gas flow tubes 107; 108; 109 may be 38 °C.

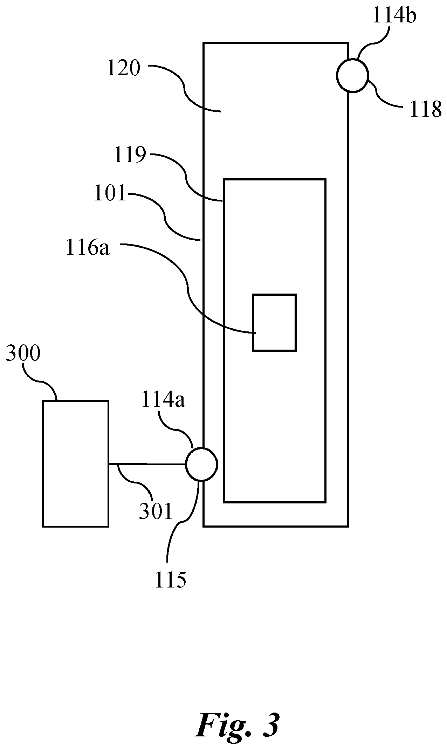

The alcohol breath gas generation device 101 in the system 100 is arranged to generate a gas mixture comprising water, air, carbon dioxide and alcohol. An alcohol gas generation device 101 is schematically illustrated in Figure 3. The alcohol gas generation device 101 comprises a gas inlet 115 at which a first valve 114a is arranged, the first valve 114a is positioned at the lower part of the alcohol breath gas generation device 101, and a gas outlet 118 arranged at the upper part of the alcohol breath gas generation device 101 where a second valve 114b is arranged. Gas is arranged to enter the alcohol breath gas generation device 101 at the first valve 114a and pass through a liquid column 119 to a dead space 120 before it exits the alcohol breath gas generation device 101 at the gas outlet 118. The gas in the dead space 120 may have a different composition than the gas that enters the gas generation device 101 at the inlet 115. The composition of the gas in the dead space 120 depends on the composition of the liquid column 119. The liquid column 119 is arranged to comprise a liquid, the liquid being a mixture of water and alcohol. During use of the system 100 gas is arranged to pass through the liquid column 119 so that a gas mixture comprising alcohol is generated in the dead space. The alcohol concentration in the gas mixture is related to the alcohol concentration in the liquid column 119, hence by varying the alcohol concentration in the liquid column 119 the alcohol concentration in the gas mixture may be varied.

The liquid in the liquid column 119 may be arranged to be saturated with CO₂. During use of the system 100 gas is arranged to enter the alcohol breath gas generation device 101 at the first valve 114a, the gas may be in the form of air, or CO₂, or N₂, or any mixture of those. The gas generation device 101 may be connected to a gas tank 300 at the first valve 114a via a gas inlet tube 301. The volume and the height of the liquid column 119 as well as the volume of the dead space 120 may affect the gas generation. During use of the test system 100 and/or the alcohol gas generation device 101 gas is arranged to bubble through the liquid column 119. Bubbling gas through the liquid column 119 may lead to that:
- the liquid in the liquid column 119 is saturated, or reached a constant steady state concentration of CO₂, with the gas, e.g. saturated with CO₂; and
- an equilibrium concentration of both water vapor and alcohol, and potentially air and carbon dioxide can be reached in the dead space 120.

During use of the system 100 gas in the dead space is arranged to travel further downstream the system 100 to reach either the outlet 110, the test chamber 103, or the reference measuring device 104. In one embodiment the volume of the dead space 120 is 0.1-3.5 L. A large volume of the dead space 120 may enable that aerosols formed in the alcohol gas generation device 101 can fall out in the dead space 120 and thus not travel with the gas further in the system 100. The absence of aerosols in the gas mixture may enable a more stable gas composition, i.e. that the concentrations of the gas components does not vary.

In one embodiment the gas flow in the system 100 may be 2 L/s, or 20 L/min, or around 0.5 L/s. The gas flow level may depend on the volume of the liquid column 119, the height of the liquid column 119, the volume of the dead space 120, etc. In one embodiment the volume of the liquid 119 is 2000-3000 ml and the height of the liquid column 119 is 200-400 mm. The gas flow in the system 100 may be measured as input flow.

In one embodiment the liquid in the liquid column 119 is arranged to be mixture of water and alcohol, and the mixture is arranged to be heated to 34 °C and bubbled with air, or air enriched with carbon dioxide (CO₂). The gas generation device 101 may be arranged to generate a gas mixture in the dead space 120 comprising alcohol, water, and CO₂.

In one embodiment the alcohol breath gas generation device 101 comprises more than one liquid column 119, such as two liquid columns, or three liquid columns or more. Each liquid column 119 may have its own inlet 115, outlet 118, heater 116 and dead volume 120. More than one liquid column 119 enables that the test system 100 may be arranged to generate gas mixtures with different concentrations without changing the liquid in the liquid column 119. Hence, each liquid column 119 may be arranged to comprise a different liquid composition that for example have different alcohol concentrations so that gas formed in the dead volume 120 above respective liquid column 119 have different compositions.

The gas that enters the liquid column 119 at the gas inlet 115 may be arranged to be dispersed so that it is in the form of small bubbles, i.e. with a diameter of 0.3-6.5 mm. Small bubbles may facilitate a more homogenous mixture of the gas with the liquid in the liquid column 119 so that the different concentrations may reach equilibrium before the gas reach the dead volume 120.

The reference measuring device 104 is arranged downstream the distribution unit 102 and the gas generation device 101. The reference measuring device 104 is arranged to measure the composition of the gas generated by the gas generation device 101. During a test sequence or method of testing the reference measuring device 104 is arranged to verify the composition of the gas. The reference measuring device 104 may be a reference instrument based on IR detection. The reference measuring device 104 may comprise a reference valve 104' that can be opened to the ambient air. The reference valve 104' may enable that the reference measuring device 104 can be arranged to flush itself during use of the test system 100. The measurements by the reference measuring device 104 may be used for comparison with the measured values from the alcohol breath analyzer unit 111. During a test sequence or method of testing, both the reference measuring device 104 and the alcohol breath analyzer unit 111 are arranged to be exposed for gas with essentially the same composition. Both the reference measuring device 104 and the alcohol breath analyzer unit 111 measures the alcohol concentration in the gas and the results may be compared for example to determine the performance of the alcohol breath analyzer unit 111. The reference measuring device 104 may be an instrument that fulfill the requirements on evidential instruments set for example in the Scandinavian countries. It may be a commercially available instrument that is verifiable. The reference measuring device 104 may be based on IR detection and operate according to Beer Lambert's law, in such case in order to verify the reference measuring device 104 it is only necessary to verify that the relative attenuation at each of the filter wavelengths is the same as it was at the time for calibration. Such a reference measuring device 104 may be an evidential breath analyzer, for example Evidenzer, Nanopuls AB, Uppsala, Sweden, with a documented measuring accuracy and precision exceeding that of standard breath analyzers. The accuracy and precision of the reference measuring device 104 may repeatedly be verified by gravimetric methodology.

The test system 100 according to the invention is controlled via the control and registration unit 105 which also is configured to collect test data. The control and registration unit 105 typically comprises a processor 500 and at least one storage unit 510 for storing the program sequences and collected data and an I/O-unit for communication with a multi-purpose computer, for example. The control and registration unit 105 is functionally connected to the first valve 114a via a first functional signal pathway 501, the second valve 114b via a second functional pathway 502, the distribution unit 102 comprising the third 114c, fourth 114d, and fifth valve 114e, via a third functional signal pathway 503, the reference measuring device 104 via a fourth functional signal pathway 504, the test chamber 103 via a fifth functional signal pathway 505, and to the at least one alcohol breath analyzer unit 111 via a sixth functional signal pathway 506. As realized by the skilled person the hardware of the control and registration unit 105 and the connection to other units in the test system 100 may be realized in a number of ways, for example the connections being directly wired, via a bus-system or wireless. Similarly, the architecture of a control and registration unit 105 may vary and the described units should be regarded as functional units. Suitable multi-purpose control and registration units suitable for the test system according to the invention are commercially available, for example.

A test system 100 according to the invention may enable at least one alcohol breath analyzer unit 111 to be tested using gas with an alcohol concentration that has a variation of less than 0.5 µg/L, or less than 0.1 µg/L. For a test system to be reliable it is important that the concentration of the gas composition, for example the alcohol concentration in the gas does not show a large variation. A test system 100 according to the invention, or a gas generation device 101 may generate a gas composition wherein the alcohol concentration is 0-5 mg/L, or 0-2.5 mg/L.

The valves 114a-e in the system 100 can be automated in order to regulate the flow of gas through the system 100. The control and registration unit 105 may be configured to provide such a regulation of the valves 114a-e.

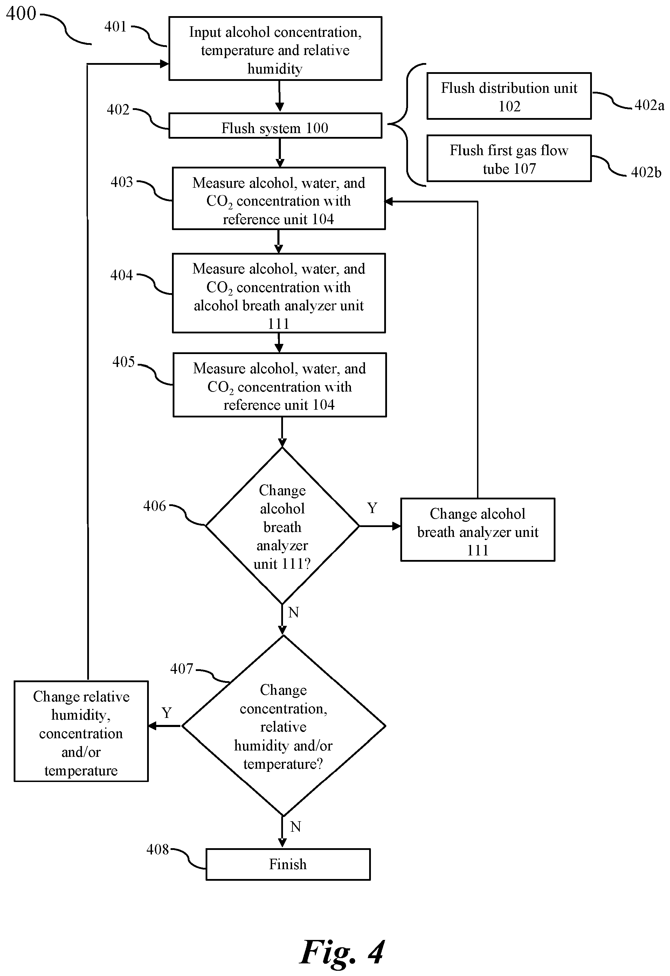

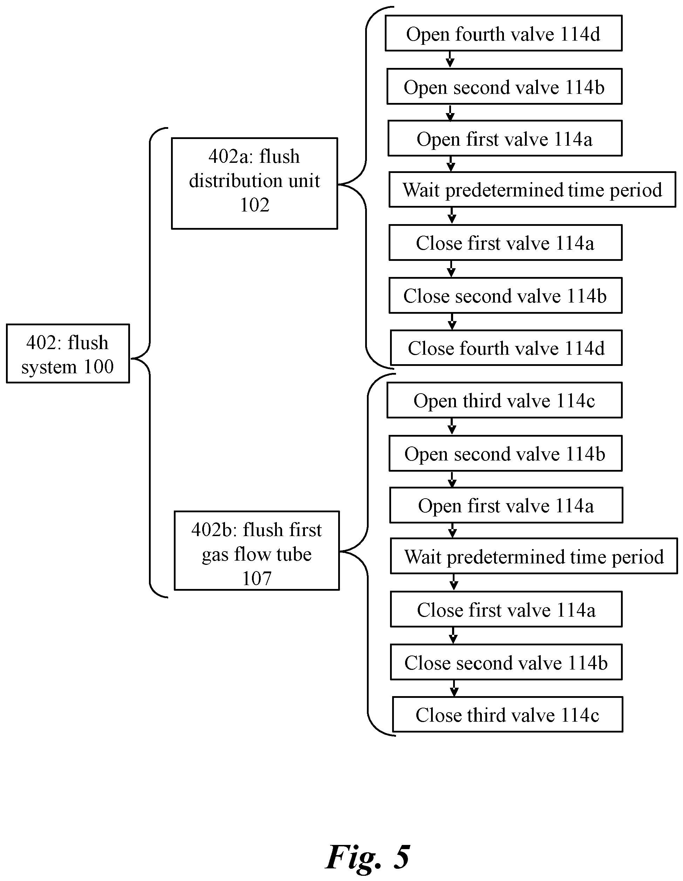

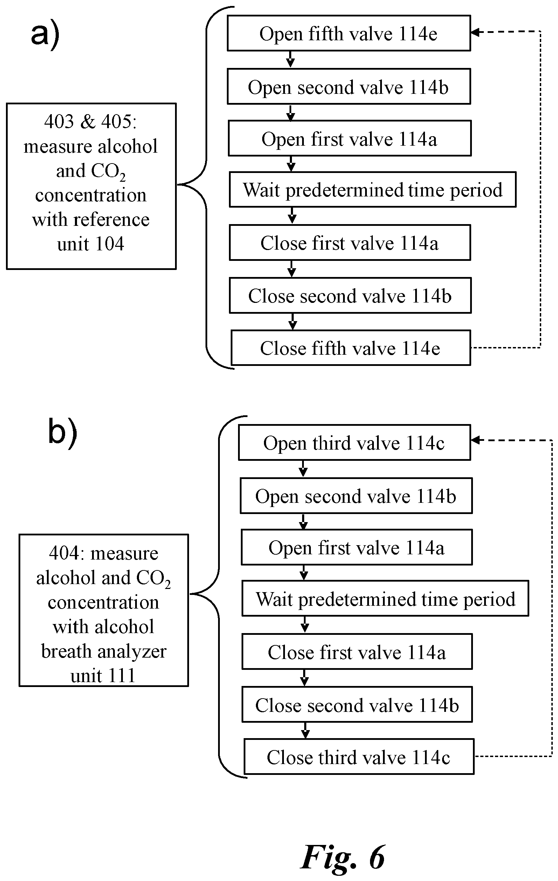

The at least one alcohol breath analyzer unit 111 may be tested for performance by the system 100, e.g. how accurate the alcohol breath analyzer unit 111 can measure the concentrations of alcohol, CO₂, and possible other gases in a gas mixture. Such a test is performed by exposing the at least one alcohol breath analyzer unit 111 for gas generated by the system 100 and/or gas generation device 101. The at least one alcohol breath analyzer unit 111 is exposed to the gas in a method of testing 400, illustrated in Figure 4. During the method of testing 400 the at least one alcohol breath analyzer unit 111 is arranged in the test chamber 103. Prior to starting a method of testing 400 all valves 114a-e in the system 100 are closed. The method of testing 400 comprises the following steps:
**401:** input values for temperature, relative humidity, alcohol concentration to the system 100;
**402:** flush system 100, step 402 comprises the substeps:
   **402a:** flush system via the third gas flow path 123, this step may ensure that the distribution unit 102 comprises the alcohol concentration inputted in step 401. To perform step 402a the fourth valve 114d is opened first, followed by the second valve 114b, and last the first valve 114a. Once the first valve 114a is opened gas is transported in the system 100 via the third gas flow path 123 and exits the system at the outlet 110. After a pre-determined time period the first valve 114a, second valve 114b, and fourth valve 114d that are open are closed again in the reverse order, i.e. the first valve 114a is closed first, followed by the second valve 114b, and last the fourth valve 114d is closed. The opening and closing order of the valves 114b; 114d; 114a in step 402 is schematically illustrated in Figure 5; and
   **402b:** flush system 100 via the first gas flow path 121, this step may ensure that the first gas flow tube 107 comprises the alcohol concentration inputted in step 401. In step 402b the third valve 114c is opened first, followed by the second valve 114b, and last the first valve 114a. Once the first valve 114a is opened gas is transported in the system 100 via the first gas flow path 121 and enters the test chamber 103 that has an opening 103' to the ambient air. After a pre-determined time period the first 114a, second 114b, and third 114c valves that are open are closed again in the reverse order, i.e. the first valve 114a is closed first, followed by the second valve 114b, and last the third valve 114c is closed. The opening and closing order of the valves 114c; 114b; 114a in step 402 is schematically illustrated in Figure 5.
**403:** measure alcohol concentration and possible CO₂ and water concentration with reference unit 104. To measure the alcohol and CO₂ concentration of the gas with the reference unit 104 the reference unit 104 is exposed to gas via the second gas flow path 122. To enable the measurement the fifth valve 114e is opened, followed by the second valve 114b, and last the first valve 114a. Once the first valve 114a is opened gas is transported in the system 100 via the second gas flow path 122 and the reference unit 104 is exposed to the gas for a predetermined time period of for example 2-4 seconds. After the exposure the fifth 114e, second 114b, and first 114a valve that are open are closed again in the reverse order, i.e. the first valve 114a is closed first followed by the second valve 114b, and last the fifth valve 114e. Step 403 may be repeated one or several times, so that the reference unit 104 can be exposed for gas a repeated number of times. The opening and closing order of the valves 114e; 114b; 114a in step 403 is schematically illustrated in Figure 6a;
**404:** measure alcohol and possible CO₂ concentration with the at least one alcohol breath analyzer unit 111 arranged in the test chamber 103. To measure the alcohol and CO₂ concentration of the gas with the at least one alcohol breath analyzer unit 111, the at least one alcohol breath analyzer unit 111 is exposed for gas via the first gas flow path 121. To enable the measurement the third valve 114c is opened, followed by the second valve 114b, and last the first valve 114a. Once the first valve 114a is opened gas is transported in the system 100 via the first gas flow path 121 and the at least one alcohol breath analyzer unit 111 is exposed to the gas for a predetermined time period of for example 2-4 seconds. After the exposure the third 114c, second 114b, and first 114a valve that are open are closed again in the reverse order, i.e. the first valve 114a is closed first followed by the second valve 114b and last the third valve 114c. Step 404 may repeated one or several times, such as ten times for example so that the at least one alcohol breath analyzer unit 111 is exposed to gas a repeated number of times. The opening and closing order of the valves 114c; 114b; 114a in step 403 is schematically illustrated in Figure 6b;
**405:** measure alcohol concentration with reference unit 104. Step 405 is a repetition of step 403. Hence, the fifth valve 114e is opened, followed by the second valve 114b, and last the first valve 114a. Once the first valve 114a is opened gas is transported in the system 100 via the third gas flow path 123 and the reference unit 104 is exposed to the gas for a predetermined time period of for example 2-4 seconds. After the exposure the fifth 114e, second 114b, and first 114a valve that are open are closed again in the reverse order, i.e. the first valve 114a is closed first followed by the second valve 114b and last the fifth valve 114e. Step 405 may repeated one or several times, such as ten times for example so that the reference unit 104 is exposed to gas a repeated number of times. The opening and closing order of the valves 114e; 114b; 114a in step 403 is schematically illustrated in Figure 6a.

The method may comprise the steps 401 of inputting values to the system 100, 402 of flushing the system 100, 403 of measuring with the reference measuring device 104, and 404 of measuring with the alcohol breath analyzer unit 111. In such case step 403 of measuring the alcohol concentration with the reference unit 104 may be performed prior to, or after, step 404 of measuring the alcohol concentration with the at least one alcohol breath analyzer unit 111.

The reference unit 104 may comprise a reference valve 104' that can be opened to the ambient air. In such case the reference unit 104 may flush itself in between, or during, methods of testing 400.

In case that more than one alcohol breath analyzer unit 111 are to be tested, hence that the test chamber 103 comprises more than one alcohol breath analyzer unit, the method of testing 400 may comprise an additional step 406 after step 405, in which the alcohol breath analyzer unit 111 is changed. Such a change may be performed by the device for changing alcohol breath analyzer unit 200. In a case when the method of testing 400 comprises step 406, step 406 is followed by steps 403, 404, and 405.

The method of testing 400 may comprise a step 407 in which the conditions of the testing can be changed, i.e. the relative humidity and/or temperature of the test chamber 103, and/or the concentration of alcohol. If any of the conditions are changed, step 407 is followed by step 401 and hence, the method of testing 400 start over.

Step 402 of flushing the system 100 may ensure that no residues are left in the system 100 from the previous gas concentration.

The control and registration unit 105 may be configured to control the method of testing 400 so that the control and registration unit 105 is configured to transmit signals to the valves 114a-e in the system to control when they are to be opened or closed. Step 401 of inputting alcohol concentration, temperature, and relative humidity may be performed by inputting such data to the control and registration unit 105 that may be configured to use such data to control the opening and closing of valves 114a-e in the system 100 during a method of testing 400. The control and registration unit 105 may further be configured to use such inputted data to control the relative humidity and/or temperature of the test chamber 103, as well as the temperature in the system 100 for example the temperature of the first 116a, and second 116b heater.

In all steps of the method of testing 400 when the first valve 114a is opened gas enters from the gas tank 300 into the system 100. The gas enters the liquid column 119 that comprises water and alcohol, or only water in the case that the zero level is to be tested. The concentration of alcohol in the liquid column 119 may be manually regulated or regulated using another suitable mean. When gas enters the liquid column 119 it is mixed with the liquid and a gas comprising water, air, carbon dioxide, and possible alcohol is generated in the dead space 120. The generated gas may have a controlled concentration of alcohol and/or carbon dioxide. The gas tank 300 may have an internal pressure that is higher than the internal pressure of the system 100, therefore the first valve 114a should preferably be opened last and closed first in a method where the valves 114a-e in the system 100 are opened and closed so that the first valve 114a is not open when the other valves, i.e. the second 114b, third 114c, fourth 114d, and fifth valve 114e are closed.

In one embodiment the method of testing 400 may comprise the following steps:
**410:** define the first 121, second 122, and third 123 gas flow path from the common starting point, i.e. the first valve 114a, to the respective endpoints, i.e. the test chamber 103 for the first gas flow path 121, the outlet 110 for the second gas flow path 122, and the reference measuring unit 104 for the third gas flow path 123. Each gas flow path 121; 122; 123 is provided with a plurality of valves, the first valve 114a is common to all gas flow paths 121; 122; 123;
**411:** opening the first valve 114a to provide gas to an endpoint of one of the three gas flow paths 121; 122; 123, in a sequence starting with the outermost downstream valve and consecutively open the plurality of valves in one of the first 121, second 122, or third 123 gas flow path in the upstream direction finishing with the first common valve 114a; and
**412:** ending the provision of gas to an endpoint by closing the valves of a gas flow path 121; 122; 123 starting with the first common valve 114a and consecutively close the plurality of valves in the gas flow path 121; 122; 123 in the downstream direction finishing with the valve closest to the endpoint, i.e. the third valve 114c for the first gas flow path 121, the fifth valve 114e for the second gas flow path 122, and the fourth valve 114d for the third gas flow path 123.

As discussed above a control and registration unit 105 is arranged to control the system 100 and to register the results from the alcohol concentration measurements. The control and registration unit 105 may record data such as power on time, startup time, CO₂ concentration, alcohol concentration, temperature, voltages, currents, etc. The control and registration unit 105 may be arranged to control the temperature of the gas generation device 101, the distribution unit 102, and the test chamber 103. The control and registration unit 105 may further be arranged to store data and generate reports that comprises temperature, relative humidity, concentration levels in the tank 300, measurement data from the alcohol breath analyzer unit 111 and reference unit 104, etc. The control and registration unit 105 may further be arranged to control the valves 114a-e inside the system 100. As described in the method of testing 400, for example in steps 402, 403, 404 and 405 above, the valves 114a-e may be controlled so that they are opened from the outside and in, i.e. the third 114c, fifth 114e, and fourth 114d valve furthest downstream the system 100 are opened first, followed by the second valve 114b in between the distribution unit 102 and the gas generation device 101, and at last the first valve 114a arranged at the gas generation device 101 is opened. Such an opening sequence of the valves 114a-e may enable the system 100 to have the same, or almost the same, pressure as the surrounding atmosphere. The valves 114a-e may further be controlled by the control and registration unit 105 so that when they are closed they are closed in the reverse order from the inside and out, hence the first valve 114a arranged at the gas generation device 101 is closed first, followed by the second valve 114b. after which one or all of the third 114c, fourth 114d, and fifth valve 114e are closed.

All aspects and embodiments can be combined with each other unless explicitly stated otherwise.

## Claims

1. A system (100) for testing at least one alcohol breath analyzer (111) comprising:
an alcohol breath gas generation device (101) comprising at least one liquid column (119), wherein the gas generation device (101) comprises a gas inlet (115) arranged at the lower part of the gas generation device (101) and a gas outlet (118) arranged at the upper part of the gas generation device (101),
wherein gas enters the gas generation device (101) at the gas inlet (115), mixes with the liquid in the liquid column (119), and exits the gas generation device (101) at the gas outlet (118);
a test chamber (103) in fluid communication with the alcohol breath gas generation device (101), wherein the test chamber (103) is arranged to receive at least one alcohol breath analyzer (111);
wherein the liquid in the at least one liquid column (119) comprises water and a pre-determined concentration of alcohol, and comprising a control and registration unit (105) arranged to control the parts of the alcohol breath analyzer system (100) and to register the testing results,**characterized in that** it comprises:
a reference measuring device (104) in fluid communication with the alcohol breath gas generation device (101), the reference measuring device (104) being configured to measure the concentration of alcohol in the gas exiting the gas outlet (118) of the gas generation device (101);
and **in that** the system (100) further comprises:
a distribution unit (102) in fluid communication with the gas generation device (101), the distribution unit (102) arranged to distribute gas from the gas generation device (101) to one of three gas flow paths: a first gas flow path (121) arranged to distribute gas from the gas generation device (101) to the test chamber (103), a second gas flow path (122) arranged to distribute gas from the gas generation device (101) to the reference measuring device (104), and a third gas flow path (123) arranged to distribute gas from the gas generation device (101) to an opening to the ambient air.

2. The test system (100) according to claim 1 wherein the system further comprises valve means arranged to shift the gas flow between the three gas flow paths: the first gas flow path (121), the second gas flow path (122), and the third gas flow path (123).

3. The test system (100) according to claim 1 or 2, wherein the third gas flow path (123) is arranged to distribute gas from the gas generation device (101) to an outlet (110).

4. The test system (100) according to any of claims 1-3, wherein the test system comprises a first valve (114a) arranged at the inlet (115) at the gas generation device (101), arranged to regulate the gas flow into the gas generation device (101), a second valve (114b) arranged in between the gas generation device (101), and the distribution unit (102), the second valve (114b) is arranged to regulate the gas flow from the gas generation device (101) to the distribution unit (102), a third valve (114c), a fourth valve (114d), and a fifth valve (114e) arranged at the distribution unit (102), the third valve (114c) is arranged to regulate the gas flow into the first gas tube (107), the fourth valve (114d) is arranged to regulate the gas flow into the third gas tube (109), and the fifth valve (114e) is arranged to regulate the gas flow into the second gas tube (108), wherein the first valve (114a), the second valve (114b), and the third valve (114c) are arranged to control the gas flow in the first gas flow path (121), the first valve (114a), the second valve (114b), and the fifth valve (114e) are arranged to control the gas flow in the second gas flow path (122), and the first valve (114a), the second valve (114b), and the fourth valve (114d) are arranged to regulate the gas flow in the third gas flow path (123).

5. The test system (100) according to claim 4, wherein the inner diameter of the valves 114a-e is 0.5-2 cm.

6. The test system (100) according to any of claims 1-5, wherein the test system (100) comprises at least a first (116a) and a second (116b) heater arranged to heat the system (100) so that the temperature is higher at the distribution unit (102) than at the gas generation device (101).

7. The test system (100) according to any of claims 1-6, wherein the gas generation device (101) has a volume of 3000-7000 cm³.

8. The test system (100) according to any of claims 1-7, wherein the liquid column (119) has a volume of 2000-3000 ml.

9. The test system (100) according to any of claims 7-8, wherein the gas generation device (101) further comprises a dead space (120), and wherein the volume of the dead space (120) is 0.1-3.5 L.

10. The test system (100) according to any of claims 1-9, wherein the test system (100) further comprises a device for changing alcohol breath analyzer (200) arranged in the test chamber (103).

11. The test system (100) according to claim 10, wherein the device for changing alcohol breath analyzer (200) comprises a revolver mechanism.

12. The test system (100) according to any of the preceding claims wherein the test system (100) comprises more than one gas generation devices (101), such as two gas generation devices or three.

13. A method of testing (400) performance of at least one alcohol breath analyzer (111) using a system (100) that comprises:
an alcohol breath gas generation device (101), comprising a liquid column (119) comprising a liquid and a pre-determined concentration of alcohol, wherein the gas generation device (101) comprises a gas inlet (115) arranged at the lower part of the gas generation device (101) and a gas outlet (118) arranged at the upper part of the gas generation device (101);
a test chamber (103) in fluid communication with the alcohol breath gas generation device (101), the test chamber (103) is arranged to receive at least one alcohol breath analyzer (111);
a reference measuring device (104) in fluid communication with the alcohol breath gas generation device (101), the reference measuring device (104) being configured to measure a concentration of alcohol in a gas exiting the gas outlet (118) of the gas generation device (101);
a control and registration unit (105) arranged to control the parts of the alcohol breath analyzer system 100; and
a distribution unit (102) in fluid communication with the gas generation device (101), the distribution unit (102) arranged to distribute gas from the gas generation device (101) to one of three gas flow paths: a first gas flow path (121) arranged to distribute gas from the gas generation device (101) to the test chamber (103), a second gas flow path (122) arranged to distribute gas from the gas generation device (101) to the reference measuring device (104), and a third gas flow path (123) arranged to distribute gas from the gas generation device (101) to an outlet (110),
wherein the method of testing (400) comprises the steps of:
passing gas into the gas inlet (115) of the gas generation device (101) such that the gas mixes with the water and the pre-determined concentration of alcohol in the liquid column (119) and then exits the gas outlet (118) of the gas generation device (101);
401: inputting values for temperature, relative humidity and alcohol concentration into the system (100);
402: flushing the system (100) first via the second gas flow path (122), and then via the first gas flow path (121);
403: using the reference unit (104) to measure at least one of the alcohol, water, and C02 concentration of the gas exiting the gas outlet (118) of the gas generation device 101; and
404: measuring at least one of the alcohol, water and CO2 concentration of the at least one alcohol breath analyzer unit (111) arranged in the test chamber.

14. The method (400) according to claim 13 wherein the system (100) further comprises a first valve (114a) arranged at the inlet (115) at the gas generation device (101), a second valve (114b) arranged in between the gas generation device (101) and the distribution unit (102), a third valve (114c), a fourth valve (114d), and a fifth valve (114e) arranged at the distribution unit (102), wherein the valves 114a-e are arranged to control the gas flow in the system (100) the method comprises the steps of:
411: opening the first valve (114a) to provide gas to an endpoint of one of the three gas flow paths: the first gas flow path 121; the second gas flow path 122; or the third gas flow path (123), in a sequence starting with the outermost downstream valve and consecutively open the plurality of valves of one of the first gas flow path 121; the second gas flow path 122; or the third gas flow path (123), in the upstream direction finishing with the first common valve 114a; and
412: ending the provision of gas to an endpoint by closing the valves of one of the first gas flow path 121; the second gas flow path 122; or the third gas flow path (123) starting with the first common valve (114a) and consecutively close the plurality of valves in the flow path (121; 122; 123) in the downstream direction finishing with the valve closest to the endpoint, i.e. the third valve (114c) for the first gas flow path (121), the fifth valve (114e) for the second gas flow path (122), and the fourth valve (114d) for the third gas flow path (123).

15. The method (400) according to claim 13 or 14 wherein the method (400) comprises opening of the valves 114a-e in the system (100) wherein:
- one of the third valve (114c), fourth valve (114d), and fifth valve (114e) is opened first;
- after the opening of one of the third valve (114c), fourth valve (114d), or fifth valve (114e) the second valve (114b) is opened; and
- after the opening of the second valve (114b) the first valve (114a) is opened.

16. The method according to any of claims 13-15 wherein:
Step (402) comprises the substeps of:
402a: flushing the second gas flow path (122), by opening the fourth valve (114d), followed by the second valve (114b), and last the first valve (114a), after a pre-determined time period the first valve (114a) is closed first, followed by the second valve (114b), and last the fourth valve (114d) is closed; and
402b: flushing via the first gas flow path (121), by opening the third valve (114c), followed by the second valve (114b), and last the first valve (114a), after a pre-determined time period the first valve (114a) is closed first, followed by the second valve (114b), and last the third valve (114c) is closed;
Step 403: opening the fifth valve (114e), followed by the second valve (114b), and last the first valve (114a), after a pre-determined time period the first valve (114a) is closed first followed by the second valve (114b), and last the fifth valve (114e);
and
Step 404: opening the third valve (114c), followed by the second valve (114b), and last the first valve (114a), after a pre-determined time period the first valve (114a) is closed first followed by the second valve (114b) and last the third valve (114c).

17. The method according to any of claims 13-16 wherein the step (404) is repeated at least once.

18. The method according to claim 13-17 wherein the step (404) is repeated ten times.

## Patentansprüche

1. System (100) zum Testen mindestens eines Alkoholatemanalysators (111), umfassend:
eine Alkoholatem-Gaserzeugungseinrichtung (101) mit mindestens einer Flüssigkeitssäule (119), wobei die Gaserzeugungseinrichtung (101) einen Gaseinlass (115) umfasst, der im unteren Teil der Gaserzeugungseinrichtung (101) angeordnet ist, und einen Gasauslass (118) umfasst, der im oberen Teil der Gaserzeugungseinrichtung (101) angeordnet ist,
wobei Gas in die Gaserzeugungseinrichtung (101) am Gaseinlass (115) eintritt, sich mit der Flüssigkeit in der Flüssigkeitssäule (119) mischt und die Gaserzeugungseinrichtung (101) am Gasauslass (118) verlässt;
eine Testkammer (103) in Fluidkommunikation mit der Alkoholatem-Gaserzeugungseinrichtung (101), wobei die Testkammer (103) so ausgelegt ist, dass sie mindestens einen Alkoholatemanalysator (111) aufnimmt;
wobei die Flüssigkeit in der mindestens eine Flüssigkeitssäule (119) Wasser und eine vorbestimmte Alkoholkonzentration umfasst, und umfassend eine Kontroll- und Registrierungseinheit (105), die zur Steuerung der Teile des Alkoholatemanalysatorsystems (100) und zur Registrierung der Testergebnisse ausgelegt ist, **dadurch gekennzeichnet, dass** es umfasst:
eine Referenzmesseinrichtung (104) in Fluidkommunikation mit der Alkoholatem-Gaserzeugungseinrichtung (101), wobei die Referenzmesseinrichtung (104) so konfiguriert ist, dass sie die Konzentration von Alkohol in dem Gas misst, das aus dem Gasauslass (118) der Gaserzeugungseinrichtung (101) austritt;
und dadurch, dass das System (100) ferner umfasst:
eine Verteilereinheit (102) in Fluidkommunikation mit der Gaserzeugungseinrichtung (101), wobei die Verteilereinheit (102) so ausgelegt ist, dass sie Gas von der Gaserzeugungseinrichtung (101) auf einen von drei Gasströmungswegen verteilt: einen ersten Gasströmungsweg (121), der zur Verteilung von Gas von der Gaserzeugungseinrichtung (101) zur Testkammer (103) ausgelegt ist, einen zweiten Gasströmungsweg (122), der zur Verteilung von Gas von der Gaserzeugungseinrichtung (101) zur Referenzmesseinrichtung (104) ausgelegt ist, und einen dritten Gasströmungsweg (123), der zur Verteilung von Gas von der Gaserzeugungseinrichtung (101) zu einer Öffnung zur Umgebungsluft ausgelegt ist.

2. Testsystem (100) nach Anspruch 1, wobei das System ferner Ventilmittel umfasst, die dazu ausgelegt sind, den Gasstrom zwischen den drei Gasströmungswegen zu verschieben: dem ersten Gasströmungsweg (121), dem zweiten Gasströmungsweg (122) und dem dritten Gasströmungsweg (123).

3. Testsystem (100) nach Anspruch 1 oder 2, wobei der dritte Gasströmungsweg (123) dazu ausgelegt ist, Gas von der Gaserzeugungseinrichtung (101) an einen Auslass (110) zu verteilen.

4. Testsystem (100) nach einem der Ansprüche 1 bis 3, wobei das Testsystem ein erstes Ventil (114a), das am Einlass (115) an der Gaserzeugungseinrichtung (101) angeordnet ist, das dazu ausgelegt ist, den Gasstrom in die Gaserzeugungseinrichtung (101) zu regeln, ein zweites Ventil (114b), das zwischen der Gaserzeugungseinrichtung (101) und der Verteilereinheit (102) angeordnet ist, wobei das zweite Ventil (114b) dazu ausgelegt ist, den Gasstrom von der Gaserzeugungseinrichtung (101) zur Verteilereinheit (102) zu regeln, ein drittes Ventil (114c), ein viertes Ventil (114d) und ein fünftes Ventil (114e) umfasst, die an der Verteilereinheit (102) angeordnet sind, wobei das dritte Ventil (114c) dazu ausgelegt ist, den Gasstrom in das erste Gasrohr (107) zu regeln, das vierte Ventil (114d) dazu ausgelegt ist, den Gasstrom in das dritte Gasrohr (109) zu regeln, und das fünfte Ventil (114e) dazu ausgelegt ist, den Gasstrom in das zweite Gasrohr (108) zu regeln, wobei das erste Ventil (114a), das zweite Ventil (114b) und das dritte Ventil (114c) dazu ausgelegt sind, den Gasstrom im ersten Gasströmungsweg (121) zu steuern, das erste Ventil (114a), das zweite Ventil (114b) und das fünfte Ventil (114e) dazu ausgelegt sind, den Gasstrom im zweiten Gasströmungsweg (122) zu steuern, und das erste Ventil (114a), das zweite Ventil (114b) und das vierte Ventil (114d) dazu ausgelegt sind, den Gasstrom im dritten Gasströmungsweg (123) zu regeln.

5. Testsystem (100) nach Anspruch 4, wobei der Innendurchmesser der Ventile 114a-e 0,5 bis 2 cm beträgt.

6. Testsystem (100) nach einem der Ansprüche 1 bis 5, wobei das Testsystem (100) mindestens ein erstes Heizelement (116a) und ein zweites Heizelement (116b) umfasst, die zur Erwärmung des Systems (100) ausgelegt sind, so dass die Temperatur an der Verteilereinheit (102) höher als an der Gaserzeugungseinrichtung (101) ist.

7. Testsystem (100) nach einem der Ansprüche 1 bis 6, wobei die Gaserzeugungseinrichtung (101) ein Volumen von 3000-7000 cm³ aufweist.

8. Testsystem (100) nach einem der Ansprüche 1 bis 7, wobei die Flüssigkeitssäule (119) ein Volumen von 2000-3000 ml aufweist.

9. Testsystem (100) nach einem der Ansprüche 7 bis 8, wobei die Gaserzeugungseinrichtung (101) ferner einen Totraum (120) umfasst und wobei das Volumen des Totraums (120) 0,1-3,5 l beträgt.

10. Testsystem (100) nach einem der Ansprüche 1 bis 9, wobei das Testsystem (100) ferner eine Einrichtung zum Wechseln des Alkoholatemanalysators (200) umfasst, die in der Testkammer (103) angeordnet ist.

11. Testsystem (100) nach Anspruch 10, wobei die Einrichtung zum Wechseln des Alkoholatemanalysators (200) einen Revolvermechanismus umfasst.

12. Testsystem (100) nach einem der vorhergehenden Ansprüche, wobei das Testsystem (100) mehr als eine Gaserzeugungseinrichtung (101) umfasst, wie zwei Gaserzeugungseinrichtungen oder drei.

13. Verfahren (400) zum Testen mindestens eines Alkoholatemanalysators (111) unter Verwendung eines Systems (100), das umfasst: eine Alkoholatem-Gaserzeugungseinrichtung (101) mit einer Flüssigkeitssäule (119), die eine Flüssigkeit und eine vorbestimmte Alkoholkonzentration umfasst, wobei die Gaserzeugungseinrichtung (101) einen Gaseinlass (115) umfasst, der im unteren Teil der Gaserzeugungseinrichtung (101) angeordnet ist, und einen Gasauslass (118) umfasst, der im oberen Teil der Gaserzeugungseinrichtung (101) angeordnet ist; eine Testkammer (103) in Fluidkommunikation mit der Alkoholatem-Gaserzeugungseinrichtung (101), wobei die Testkammer (103) so ausgelegt ist, dass sie mindestens einen Alkoholatemanalysator (111) aufnimmt;
eine Referenzmesseinrichtung (104) in Fluidkommunikation mit der Alkoholatem-Gaserzeugungseinrichtung (101), wobei die Referenzmesseinrichtung (104) so konfiguriert ist, dass sie eine Konzentration von Alkohol in einem Gas misst, das aus dem Gasauslass (118) der Gaserzeugungseinrichtung (101) austritt;
eine Kontroll- und Registrierungseinheit (105), die zur Steuerung der Teile des Alkoholatemanalysatorsystems (100) ausgelegt ist; und
eine Verteilereinheit (102) in Fluidkommunikation mit der Gaserzeugungseinrichtung (101), wobei die Verteilereinheit (102) so ausgelegt ist, dass sie Gas von der Gaserzeugungseinrichtung (101) auf einen von drei Gasströmungswegen verteilt: einen ersten Gasströmungsweg (121), der dazu ausgelegt ist, Gas von der Gaserzeugungseinrichtung (101) zur Testkammer (103) zu verteilen, einen zweiten Gasströmungsweg (122), der dazu ausgelegt ist, Gas von der Gaserzeugungseinrichtung (101) zur Referenzmesseinrichtung (104) zu verteilen, und einen dritten Gasströmungsweg (123), der dazu ausgelegt ist, Gas von der Gaserzeugungseinrichtung (101) zu einem Auslass (110) zu verteilen, wobei das Testverfahren (400) die Schritte umfasst: Leiten von Gas in den Gaseinlass (115) der Gaserzeugungseinrichtung (101), so dass sich das Gas mit dem Wasser und der vorbestimmten Alkoholkonzentration in der Flüssigkeitssäule (119) mischt und dann aus dem Gasauslass (118) der Gaserzeugungseinrichtung (101) austritt;
401: Eingeben von Werten für Temperatur, relative Luftfeuchtigkeit und Alkoholkonzentration in das System (100);
402: Spülen des Systems (100) zuerst über den zweiten Gasströmungsweg (122) und dann über den ersten Gasströmungsweg (121);
403: Verwenden der Referenzeinheit (104) zur Messung von mindestens einer der Alkohol-, Wasser- und C02-Konzentration des Gases, das aus dem Gasauslass (118) der Gaserzeugungseinrichtung 101 austritt, und
404: Messen von mindestens einer der Alkohol-, Wasser- und CO2-Konzentration der mindestens einen in der Testkammer angeordneten Alkoholatemanalysatoreinheit (111).

14. Verfahren (400) nach Anspruch 13, wobei das System (100) ferner ein erstes Ventil (114a), das am Einlass (115) an der Gaserzeugungseinrichtung (101) angeordnet ist, ein zweites Ventil (114b), das zwischen der Gaserzeugungseinrichtung (101) und der Verteilereinheit (102) angeordnet ist, ein drittes Ventil (114c), ein viertes Ventil (114d) und ein fünftes Ventil (114e) umfasst, die an der Verteilereinheit (102) angeordnet sind, wobei die Ventile 114a-e dazu ausgelegt sind, den Gasstrom im System (100) zu steuern, wobei das Verfahren die Schritte umfasst:
411: Öffnen des ersten Ventils (114a), um Gas zu einem Endpunkt eines der drei Gasströmungswege bereitzustellen: dem ersten Gasströmungsweg 121; dem zweiten Gasströmungsweg 122; oder dem dritten Gasströmungsweg (123), in einer Abfolge, beginnend mit dem äußersten nachgeschalteten Ventil und nacheinander Öffnen der Mehrzahl von Ventilen eines des ersten Gasströmungswegs 121; des zweiten Gasströmungswegs 122; oder des dritten Gasströmungswegs (123), in stromaufwärts gerichteter Richtung, wobei mit dem ersten gemeinsamen Ventil 114a geendet wird; und
412: Beenden der Gasbereitstellung zu einem Endpunkt durch Schließen der Ventile eines des ersten Gasströmungswegs 121; des zweiten Gasströmungswegs 122; oder des dritten Gasströmungswegs (123), beginnend mit dem ersten gemeinsamen Ventil (114a) und nacheinander Schließen der Mehrzahl von Ventilen im Strömungsweg (121; 122; 123) in nachgeschalteter Richtung, wobei mit dem Ventil geendet wird, das dem Endpunkt am nächsten liegt, d. h. dem dritten Ventil (114c) für den ersten Gasströmungsweg (121), dem fünften Ventil (114e) für den zweiten Gasströmungsweg (122) und dem vierten Ventil (114d) für den dritten Gasströmungsweg (123).

15. Verfahren (400) nach Anspruch 13 oder 14, wobei das Verfahren (400) das Öffnen der Ventile 114a-e in dem System (100) umfasst, wobei:
- eines des dritten Ventils (114c), des vierten Ventils (114d) und des fünften Ventils (114e) zuerst geöffnet wird;
- nach dem Öffnen eines des dritten Ventils (114c), des vierten Ventils (114d) oder des fünften Ventils (114e) das zweite Ventil (114b) geöffnet wird; und
- nach dem Öffnen des zweiten Ventils (114b) das erste Ventil (114a) geöffnet wird.

16. Verfahren nach einem der Ansprüche 13 bis 15, wobei:
Schritt (402) die Teilschritte umfasst:
402a: Spülen des zweiten Gasströmungswegs (122) durch Öffnen des vierten Ventils (114d), gefolgt vom zweiten Ventil (114b) und zuletzt dem ersten Ventil (114a), wobei nach einer vorbestimmten Zeitspanne das erste Ventil (114a) zuerst geschlossen wird, gefolgt vom zweiten Ventil (114b), und zuletzt wird das vierte Ventil (114d) geschlossen; und
402b: Spülen über den ersten Gasströmungsweg (121) durch Öffnen des dritten Ventils (114c), gefolgt vom zweiten Ventil (114b) und zuletzt dem ersten Ventil (114a), wobei nach einer vorbestimmten Zeitspanne das erste Ventil (114a) zuerst geschlossen wird, gefolgt vom zweiten Ventil (114b), und zuletzt wird das dritte Ventil (114c) geschlossen;
Schritt 403: Öffnen des fünften Ventils (114e), gefolgt vom zweiten Ventil (114b) und zuletzt dem ersten Ventil (114a), wobei nach einer vorbestimmten Zeitspanne das erste Ventil (114a) zuerst geschlossen wird, gefolgt vom zweiten Ventil (114b) und zuletzt dem fünften Ventil (114e);und
Schritt 404: Öffnen des dritten Ventils (114c), gefolgt vom zweiten Ventil (114b) und zuletzt dem ersten Ventil (114a), wobei nach einer vorbestimmten Zeitspanne das erste Ventil (114a) zuerst geschlossen wird, gefolgt vom zweiten Ventil (114b) und zuletzt dem dritten Ventil (114c).

17. Verfahren nach einem der Ansprüche 13 bis 16, wobei Schritt (404)mindestens einmal wiederholt wird.

18. Verfahren nach Anspruch 13 bis 17, wobei Schritt (404) zehnmal wiederholt wird.

## Revendications

1. Système (100) pour tester au moins un éthylotest (111) comprenant :
un dispositif de génération de gaz d'haleine alcoolisée (101) comprenant au moins une colonne de liquide (119), dans lequel le dispositif de génération de gaz (101) comprend une entrée de gaz (115) agencée à la partie inférieure du dispositif de génération de gaz (101) et une sortie de gaz (118) agencée à la partie supérieure du dispositif de génération de gaz (101),
dans lequel du gaz pénètre dans le dispositif de génération de gaz (101) au niveau de l'entrée de gaz (115), se mélange avec le liquide dans la colonne de liquide (119), et sort du dispositif de génération de gaz (101) au niveau de la sortie de gaz (118) ;
une chambre de test (103) en communication fluidique avec le dispositif de génération de gaz d'haleine alcoolisée (101), dans lequel la chambre de test (103) est agencée pour recevoir au moins un éthylotest (111) ;
dans lequel le liquide dans l'au moins une colonne de liquide (119) comprend de l'eau et une concentration prédéterminée d'alcool, et comprenant une unité de commande et d'enregistrement (105) agencée pour commander les parties du système d'éthylotest (100) et pour enregistrer les résultats de test, **caractérisé en ce qu'**il comprend :
un dispositif de mesure de référence (104) en communication fluidique avec le dispositif de génération de gaz d'haleine alcoolisée (101), le dispositif de mesure de référence (104) étant configuré pour mesurer la concentration d'alcool dans le gaz sortant de la sortie de gaz (118) du dispositif de génération de gaz (101) ;
et **en ce que** le système (100) comprend en outre :
une unité de distribution (102) en communication fluidique avec le dispositif de génération de gaz (101), l'unité de distribution (102) étant agencée pour distribuer du gaz provenant du dispositif de génération de gaz (101) vers l'un de trois chemins d'écoulement de gaz : un premier chemin d'écoulement de gaz (121) agencé pour distribuer du gaz provenant du dispositif de génération de gaz (101) vers la chambre de test (103), un deuxième chemin d'écoulement de gaz (122) agencé pour distribuer du gaz provenant du dispositif de génération de gaz (101) vers le dispositif de mesure de référence (104), et un troisième chemin d'écoulement de gaz (123) agencé pour distribuer du gaz provenant du dispositif de génération de gaz (101) vers une ouverture sur l'air ambiant.

2. Système de test (100) selon la revendication 1, dans lequel le système comprend en outre des moyens de valve agencés pour commuter l'écoulement de gaz entre les trois chemins d'écoulement de gaz : le premier chemin d'écoulement de gaz (121), le deuxième chemin d'écoulement de gaz (122), et le troisième chemin d'écoulement de gaz (123).

3. Système de test (100) selon la revendication 1 ou 2, dans lequel le troisième chemin d'écoulement de gaz (123) est agencé pour distribuer du gaz provenant du dispositif de génération de gaz (101) vers une sortie (110).

4. Système de test (100) selon l'une quelconque des revendications 1 à 3, dans lequel le système de test comprend une première valve (114a) agencée au niveau de l'entrée (115) au niveau du dispositif de génération de gaz (101), agencée pour réguler l'écoulement de gaz dans le dispositif de génération de gaz (101), une deuxième valve (114b) agencée entre le dispositif de génération de gaz (101) et l'unité de distribution (102), la deuxième valve (114b) est agencée pour réguler l'écoulement de gaz provenant du dispositif de génération de gaz (101) vers l'unité de distribution (102), une troisième valve (114c), une quatrième valve (114d), et une cinquième valve (114e) agencées au niveau de l'unité de distribution (102), la troisième valve (114c) est agencée pour réguler l'écoulement de gaz dans le premier tube de gaz (107), la quatrième valve (114d) est agencée pour réguler l'écoulement de gaz dans le troisième tube de gaz (109), et la cinquième valve (114e) est agencée pour réguler l'écoulement de gaz dans le deuxième tube de gaz (108), dans lequel la première valve (114a), la deuxième valve (114b), et la troisième valve (114c) sont agencées pour commander l'écoulement de gaz dans le premier chemin d'écoulement de gaz (121), la première valve (114a), la deuxième valve (114b), et la cinquième valve (114e) sont agencées pour commander l'écoulement de gaz dans le deuxième chemin d'écoulement de gaz (122), et la première valve (114a), la deuxième valve (114b), et la quatrième valve (114d) sont agencées pour réguler l'écoulement de gaz dans le troisième chemin d'écoulement de gaz (123).

5. Système de test (100) selon la revendication 4, dans lequel le diamètre intérieur des valves 114a-e est de 0,5 à 2 cm.

6. Système de test (100) selon l'une quelconque des revendications 1 à 5, dans lequel le système de test (100) comprend au moins un premier (116a) et un deuxième (116b) dispositif de chauffage agencés pour chauffer le système (100) de sorte que la température soit plus élevée au niveau de l'unité de distribution (102) qu'au niveau du dispositif de génération de gaz (101).

7. Système de test (100) selon l'une quelconque des revendications 1 à 6, dans lequel le dispositif de génération de gaz (101) a un volume de 3000 à 7000 cm³.

8. Système de test (100) selon l'une quelconque des revendications 1 à 7, dans lequel la colonne de liquide (119) a un volume de 2000 à 3000 ml.

9. Système de test (100) selon l'une quelconque des revendications 7 à 8, dans lequel le dispositif de génération de gaz (101) comprend en outre un espace mort (120), et dans lequel le volume de l'espace mort (120) est de 0,1 à 3,5 L.

10. Système de test (100) selon l'une quelconque des revendications 1 à 9, dans lequel le système de test (100) comprend en outre un dispositif pour changer d'éthylotest (200) agencé dans la chambre de test (103).

11. Système de test (100) selon la revendication 10, dans lequel le dispositif pour changer d'éthylotest (200) comprend un mécanisme de revolver.

12. Système de test (100) selon l'une quelconque des revendications précédentes, dans lequel le système de test (100) comprend plus d'un dispositif de génération de gaz (101), tel que deux dispositifs de génération de gaz ou trois.

13. Procédé de test (400) des performances d'au moins un éthylotest (111) utilisant un système (100) qui comprend :
un dispositif de génération de gaz d'haleine alcoolisée (101), comprenant une colonne de liquide (119) comprenant un liquide et une concentration prédéterminée d'alcool, dans lequel le dispositif de génération de gaz (101) comprend une entrée de gaz (115) agencée à la partie inférieure du dispositif de génération de gaz (101) et une sortie de gaz (118) agencée à la partie supérieure du dispositif de génération de gaz (101) ;
une chambre de test (103) en communication fluidique avec le dispositif de génération de gaz d'haleine alcoolisée (101), la chambre de test (103) est agencée pour recevoir au moins un éthylotest (111) ;
un dispositif de mesure de référence (104) en communication fluidique avec le dispositif de génération de gaz d'haleine alcoolisée (101), le dispositif de mesure de référence (104) étant configuré pour mesurer une concentration d'alcool dans un gaz sortant de la sortie de gaz (118) du dispositif de génération de gaz (101) ;
une unité de commande et d'enregistrement (105) agencée pour commander les parties du système d'éthylotest (100) ; et
une unité de distribution (102) en communication fluidique avec le dispositif de génération de gaz (101), l'unité de distribution (102) étant agencée pour distribuer du gaz provenant du dispositif de génération de gaz (101) vers un de trois chemins d'écoulement de gaz : un premier chemin d'écoulement de gaz (121) agencé pour distribuer du gaz provenant du dispositif de génération de gaz (101) vers la chambre de test (103), un deuxième chemin d'écoulement de gaz (122) agencé pour distribuer du gaz provenant du dispositif de génération de gaz (101) vers le dispositif de mesure de référence (104), et un troisième chemin d'écoulement de gaz (123) agencé pour distribuer du gaz provenant du dispositif de génération de gaz (101) vers une sortie (110), dans lequel le procédé de test (400) comprend les étapes consistant à : faire passer du gaz dans l'entrée de gaz (115) du dispositif de génération de gaz (101) de telle sorte que le gaz se mélange avec l'eau et la concentration prédéterminée d'alcool dans la colonne de liquide (119) et sorte ensuite par la sortie de gaz (118) du dispositif de génération de gaz (101) ;
401 : entrer des valeurs de température, d'humidité relative et de concentration d'alcool dans le système (100) ;
402 : rincer le système (100) d'abord via le deuxième chemin d'écoulement de gaz (122), puis via le premier chemin d'écoulement de gaz (121) ;
403 : utiliser l'unité de référence (104) pour mesurer au moins une de la concentration d'alcool, d'eau et de CO2 du gaz sortant de la sortie de gaz (118) du dispositif de génération de gaz (101) ; et
404 : mesurer au moins une de la concentration d'alcool, d'eau et de CO2 de l'au moins une unité d'éthylotest (111) agencée dans la chambre de test.

14. Procédé (400) selon la revendication 13 dans lequel le système (100) comprend en outre une première valve (114a) agencée au niveau de l'entrée (115) au niveau du dispositif de génération de gaz (101), une deuxième valve (114b) agencée entre le dispositif de génération de gaz (101) et l'unité de distribution (102), une troisième valve (114c), une quatrième valve (114d), et une cinquième valve (114e) agencées au niveau de l'unité de distribution (102), dans lequel les valves 114a-e sont agencées pour commander l'écoulement de gaz dans le système (100), le procédé comprend les étapes consistant à :
411 : ouvrir la première valve (114a) pour fournir du gaz à un point d'extrémité d'un des trois chemins d'écoulement de gaz : le premier chemin d'écoulement de gaz (121) ; le deuxième chemin d'écoulement de gaz (122) ; ou le troisième chemin d'écoulement de gaz (123), selon une séquence commençant par la valve la plus à l'aval et ouvrir consécutivement la pluralité de valves de l'un du premier chemin d'écoulement de gaz (121) ; du deuxième chemin d'écoulement de gaz (122) ; ou du troisième chemin d'écoulement de gaz (123), dans la direction amont en finissant par la première valve commune (114a) ; et
412 : terminer la fourniture de gaz à un point d'extrémité en fermant les valves de l'un du premier chemin d'écoulement de gaz (121) ; du deuxième chemin d'écoulement de gaz (122) ; ou du troisième chemin d'écoulement de gaz (123) en commençant par la première valve commune (114a) et fermer consécutivement la pluralité de valves dans le chemin d'écoulement (121 ; 122 ; 123) dans la direction aval en finissant par la valve la plus proche du point d'extrémité, à savoir la troisième valve (114c) pour le premier chemin d'écoulement de gaz (121), la cinquième valve (114e) pour le deuxième chemin d'écoulement de gaz (122), et la quatrième valve (114d) pour le troisième chemin d'écoulement de gaz (123).

15. Procédé (400) selon la revendication 13 ou 14 dans lequel le procédé (400) comprend l'ouverture des valves 114a-e dans le système (100) dans lequel :
- l'une de la troisième valve (114c), de la quatrième valve (114d) et de la cinquième valve (114e) est ouverte en premier ;
- après l'ouverture de l'une de la troisième valve (114c), de la quatrième valve (114d) ou de la cinquième valve (114e), la deuxième valve (114b) est ouverte ; et
- après l'ouverture de la deuxième valve (114b), la première valve (114a) est ouverte.

16. Procédé selon l'une quelconque des revendications 13 à 15 dans lequel :
l'étape (402) comprend les sous-étapes consistant à :
402a : rincer le deuxième chemin d'écoulement de gaz (122), en ouvrant la quatrième valve (114d), suivie de la deuxième valve (114b), et enfin de la première valve (114a), après une période de temps prédéterminée, la première valve (114a) est fermée en premier, suivie de la deuxième valve (114b), et enfin la quatrième valve (114d) est fermée ; et
402b : rincer via le premier chemin d'écoulement de gaz (121), en ouvrant la troisième valve (114c), suivie de la deuxième valve (114b), et enfin de la première valve (114a), après une période de temps prédéterminée, la première valve (114a) est fermée en premier, suivie de la deuxième valve (114b), et enfin la troisième valve (114c) est fermée ;
l'étape 403 : ouvrir la cinquième valve (114e), suivie de la deuxième valve (114b), et enfin de la première valve (114a), après une période de temps prédéterminée, la première valve (114a) est fermée en premier suivie de la deuxième valve (114b), et enfin la cinquième valve (114e) ; et
l'étape 404 : ouvrir la troisième valve (114c), suivie de la deuxième valve (114b), et enfin de la première valve (114a), après une période de temps prédéterminée, la première valve (114a) est fermée en premier suivie de la deuxième valve (114b) et enfin la troisième valve (114c).

17. Procédé selon l'une quelconque des revendications 13 à 16 dans lequel l'étape (404) est répétée au moins une fois.

18. Procédé selon les revendications 13 à 17 dans lequel l'étape (404) est répétée dix fois.
